# EUROPEAN PATENT APPLICATION

(11) **EP 1 520 589 A1**
(43) Date of publication of application: **06.04.2005**
(21) Application number: 04027717.0
(22) Date of filing: 18.11.1999
(51) Int. Cl.: A61K 48/00, A61K 31/70, C07K 14/505, C12N 15/12

(54) **Erythropoietin in the treatment of domestic and livestock animals for anaemia**

(30) Priority: 17.12.1998 US 112651 P
(62) Divisional of application: 99309201.4
(71) Applicant: Pfizer Products Inc., Groton, Connecticut 06340 (US)
(72) Inventor: Krishnan, Balakrishnan Rajendra, East Lyme, CT 06333 (US); Sheppard, Michael George, North Stonington, CT 06359 (US)
(74) Representative: Murphy, Colm Damien

(57) **Abstract**

The present invention is directed to a method of treating domestic and livestock animals (particularly cats, dogs and pigs) for anemia by administering expression vectors encoding canine, feline or porcine erythropoietin. In addition, the invention encompasses pharmaceutical compositions containing the expression vectors.

SEQUENCE LISTING

<110> PFIZER PRODUCTS INC.

<120> ERYTHROPOIETIN IN THE TREATMENT OF DOMESTIC AND
      LIVESTOCK ANIMALS FOR ANEMIA

<130> PC10485A

<140>
<141>

<160> 3

<170> PatentIn Ver. 2.0

<210> 1
<211> 192
<212> PRT
<213> Canis familiaris

<400> 1
Met Gly Val His Glu Cys Pro Ala Leu Leu Leu Leu Leu Ser Leu Leu
  1               5                  10                  15

Leu Leu Pro Leu Gly Leu Pro Val Leu Gly Ala Pro Pro Arg Leu Ile
             20                  25                  30

Cys Asp Ser Arg Val Leu Glu Arg Tyr Ile Leu Glu Ala Arg Glu Ala
         35                  40                  45

Glu Asn Val Thr Met Gly Cys Ala Gln Gly Cys Ser Phe Ser Glu Asn
     50                  55                  60

Ile Thr Val Pro Asp Thr Lys Val Asn Phe Tyr Thr Trp Lys Arg Met
 65                  70                  75                  80

Asp Val Gly Gln Gln Ala Leu Glu Val Trp Gln Gly Leu Ala Leu Leu
                 85                  90                  95

Ser Glu Ala Ile Leu Arg Gly Gln Ala Leu Leu Ala Asn Ala Ser Gln
            100                 105                 110

Pro Ser Glu Thr Pro Gln Leu His Val Asp Lys Ala Val Ser Ser Leu
        115                 120                 125

Arg Ser Leu Thr Ser Leu Leu Arg Ala Leu Gly Ala Gln Lys Glu Ala
    130                 135                 140

Met Ser Leu Pro Glu Glu Ala Ser Pro Ala Pro Leu Arg Thr Phe Thr
145                 150                 155                 160

Val Asp Thr Leu Cys Lys Leu Phe Arg Ile Tyr Ser Asn Phe Leu Arg
                165                 170                 175

Gly Lys Leu Thr Leu Tyr Thr Gly Glu Ala Cys Arg Arg Gly Asp Arg
            180                 185                 190

<210> 2
<211> 192
<212> PRT
<213> Felis catus

<400> 2
Met Gly Val Arg Glu Cys Pro Ala Leu Leu Leu Leu Leu Ser Leu Leu
  1               5                  10                  15

Leu Leu Pro Leu Gly Leu Pro Val Leu Gly Ala Pro Pro Arg Leu Ile
             20                  25                  30

Cys Asp Ser Arg Val Leu Glu Arg Tyr Ile Leu Glu Ala Arg Glu Ala
         35                  40                  45

Glu Asn Val Thr Met Gly Cys Ala Glu Gly Cys Ser Phe Ser Glu Asn
     50                  55                  60

Ile Thr Val Pro Asp Thr Lys Val Asn Phe Tyr Thr Trp Lys Arg Met
 65                  70                  75                  80

Asp Val Gly Gln Gln Ala Val Glu Val Trp Gln Gly Leu Ala Leu Leu
                 85                  90                  95

Ser Glu Ala Ile Leu Arg Gly Gln Ala Leu Leu Ala Asn Ser Ser Gln
            100                 105                 110

Pro Ser Glu Thr Pro Gln Leu His Val Asp Lys Ala Val Ser Ser Leu
        115                 120                 125

Arg Ser Leu Thr Ser Leu Leu Arg Ala Leu Gly Ala Gln Lys Glu Ala
    130                 135                 140

Thr Ser Leu Pro Glu Ala Thr Ser Ala Ala Pro Leu Arg Thr Phe Thr

145                 150                 155                 160

Val Asp Thr Leu Cys Lys Leu Phe Arg Ile Tyr Ser Asn Phe Leu Arg
                165                 170                 175

Gly Lys Leu Thr Leu Tyr Thr Gly Glu Ala Cys Arg Arg Gly Asp Arg
            180                 185                 190

<210> 3
<211> 166
<212> PRT
<213> Sus scrofa

<400> 3
Ala Pro Pro Arg Leu Ile Cys Asp Ser Arg Val Leu Glu Arg Tyr Ile
  1               5                  10                  15

Leu Glu Ala Arg Glu Ala Glu Asn Val Thr Met Gly Cys Ala Glu Gly
             20                  25                  30

Cys Ser Phe Ser Glu Asn Ile Thr Val Pro Asp Thr Lys Val Asn Phe
         35                  40                  45

Tyr Thr Trp Lys Arg Met Asp Val Gly Gln Gln Ala Val Glu Val Trp
     50                  55                  60

Gln Gly Leu Ala Leu Leu Ser Glu Ala Ile Leu Arg Gly Gln Ala Leu
 65                  70                  75                  80

Leu Ala Asn Ser Ser Gln Pro Ser Glu Thr Pro Gln Leu His Val Asp
                 85                  90                  95

Lys Ala Val Ser Ser Leu Arg Ser Leu Thr Ser Leu Leu Arg Ala Leu
            100                 105                 110

Gly Ala Gln Lys Glu Ala Thr Ser Leu Pro Glu Ala Thr Ser Ala Ala
        115                 120                 125

Pro Leu Arg Thr Phe Thr Val Asp Thr Leu Cys Lys Leu Phe Arg Ile
    130                 135                 140

Tyr Ser Asn Phe Leu Arg Gly Lys Leu Thr Leu Tyr Thr Gly Glu Ala
145                 150                 155                 160

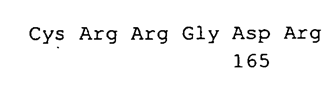

## Description

### Field of the Invention

The present invention is directed to gene therapy procedures as applied to domestic and livestock animals. In particular, the invention relates to methods and compositions for the delivery of

### Background of the Invention

Anemia caused by chronic renal failure, the administration of chemotherapeutic agents or surgery is a leading cause of death and disability in domestic and livestock animals. Apart from transfusion, the only therapy currently available for anemia in dogs, cats or pigs is the administration of recombinant human erythropoietin (EPO) protein. Although this treatment may increase the quality of life for an animal, it has two main drawbacks. First, the therapy is expensive and inconvenient (injections must typically be administered three times per week for the life of the animal). Second, treated animals often develop antibodies to the human protein that reduce its effectiveness and that may cross-react with the animal's own EPO to further exacerbate the anemia (Cowgill, *et al., J. Am. Vet. Med. Assoc. 212*:521-528 (1998)). The recent availability of canine, feline and porcine forms of EPO (Wen, *et al., Blood 82*:1507-1516 (1993)) may help to overcome the immunological effects associated with administering EPO across species lines. However, treatment is likely to remain expensive and to require frequent repetition. A method of delivering EPO that avoids these problems would represent a clear advance in veterinary medicine.

### Summary of the Invention

The present invention is based upon the discovery that anemia in dogs, cats and pigs can be alleviated by administering an expression vector encoding EPO. Such vectors are taken up by cells *in vivo* and produce sufficient hormone to significantly increase hematocrit levels in animals for a sustained period of time (3 to 4 months). By using vectors encoding the form of EPO normally found in the treated animal (canine EPO for dogs, feline EPO for cats and porcine EPO for pigs) the immunological problems associated with administering human EPO to these animals can be avoided.

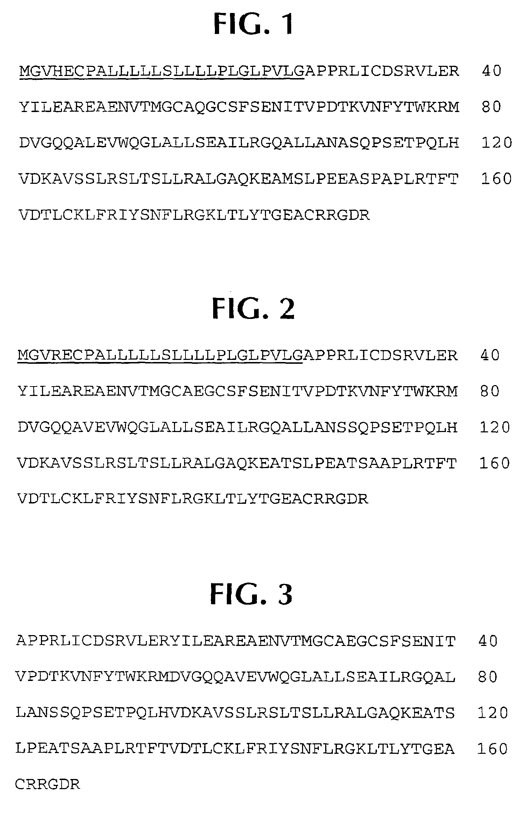

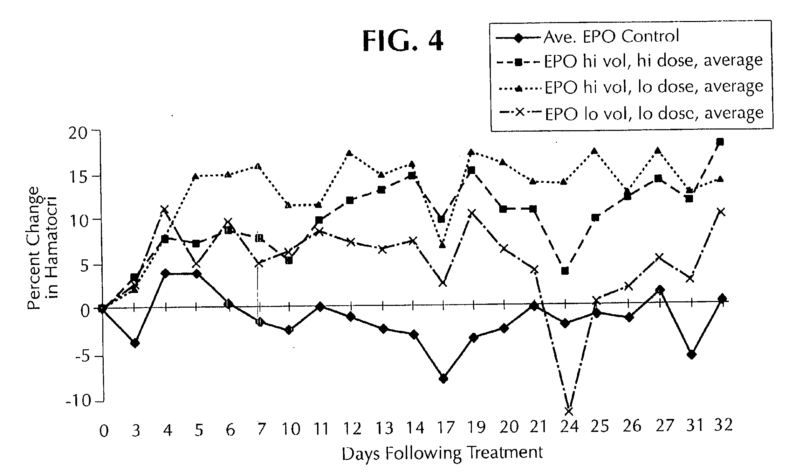

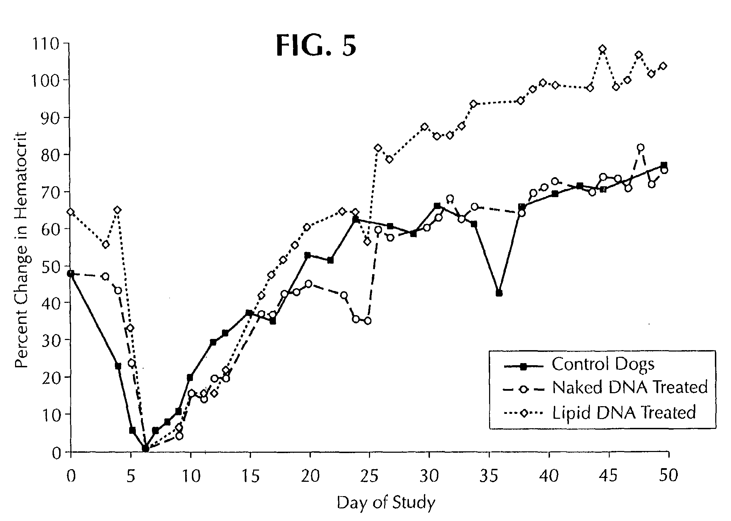

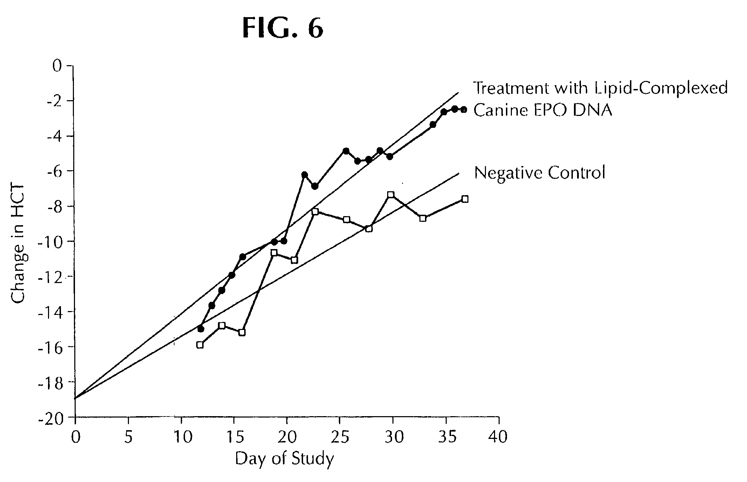

In its first aspect, the invention is directed to a method of treating anemia in dogs by administering an expression vector that has a distinct EPO sequence element consisting essentially of nucleotides encoding canine erythropoietin. The term "consisting essentially of" is intended,to encompass nucleic acid sequences encoding the exact amino acid sequence of canine EPO as well as nucleic acids encoding proteins with amino acid differences that are not substantial as evidenced by their retaining the basic functional and immunological properties of the canine hormone. It is of particular importance in this regard that the EPO encoded not induce the generation of anti-EPO antibodies in the treated animal. The EPO sequence element encodes the amino acids of the mature EPO protein, 166 residues in the case of canine EPO. Typically this will have a signal sequence (preferably a homologous signal sequence) at its N-terminal end. The term "homologous signal sequence" refers to the signal sequence normally associated with the encoded EPO, e.g., the canine EPO signal sequence would preferably be used with the canine EPO structural sequence. The complete amino acid sequence of the canine hormone is shown as Figure 1 (SEQ ID NO:1). The first 26 amino acids (underlined in the figure) represent the canine signal sequence and the remaining amino acids represent the structural sequence of mature canine EPO.

The expression vector used to treat animals should preferably have a promoter element operably linked to the EPO sequence element. The term "operably linked" means that the two sequence elements (*i.e.,* the promoter and EPO structural sequence) are joined in such a way that transcription is under the control of the promoter and the EPO protein is correctly made. The expression vector should be administered at a dosage sufficient to cause a statistically significant increase in the hematocrit of the treated animal.

A number of different agents have been described that can be used to enhance the cellular uptake of nucleic acids administered *in vivo.* Although any of these are compatible with the present invention, it is preferred that expression vectors be administered either complexed with a cationic lipid, as part of a liposome or in a form free from transfection-facilitating agents all together. The expression vector should generally be administered at a concentration of between 0.01 mg/ml and 10 mg/ml with the preferred concentration being about 0.1 mg/ml. The preferred method of delivery is by means of intramuscular injection but other methods, e.g., by means of a "gene gun," may also be used.

In another aspects of the invention, the methods for treating anemia discussed above may be applied to cats and to pigs by replacing the canine EPO sequence element in expression vectors with a feline EPO sequence element or a porcine sequence element. Thus, cats would be administered an expression vector having a distinct EPO sequence element consisting essentially of nucleotides encoding feline EPO and operably linked to a promoter element. In the case of pigs, the distinct EPO sequence element in the expression vector would consist essentially of nucleotides encoding porcine EPO. As with dogs, there should typically be a signal sequence at the N-terminal end of the encoded EPO and expression vectors should be administered at a dosage sufficient to cause a statistically significant increase in the hematocrit of the treated animal. It is preferred that the expression vectors administered be either complexed with a cationic lipid, incorporated as part of a liposome or in a form free from any transfection-facilitating agents. The sequence of feline EPO is shown in Figure 2 (SEQ ID NO:2). The underlined amino acids in the figure represent the feline signal sequence. The structural sequence of porcine EPO is shown in Figure 3 (SEQ ID NO:3).

The invention also encompasses pharmaceutical compositions for parenteral administration containing either the canine, feline or porcine expression vectors discussed above. The vectors should be suspended or dissolved in an aqueous solvent such as saline and should be present at a concentration of between about 0.01 mg/ml and 10 mg/ml. The preferred concentration is about 0.1 mg/ml.

### Brief Description of the Drawings

Figure 1: Figure 1 shows the complete amino acid sequence of canine EPO. Underlined amino acids represent the canine signal sequence and the remaining amino acids represent the structural sequence of mature canine EPO.
Figure 2: Figure 2 shows the complete amino acid sequence of feline EPO. Underlined amino acids represent the feline signal sequence and the remaining amino acids represent the structural sequence of mature feline EPO.
Figure 3: Figure 3 shows the complete structural amino acid sequence of mature porcine EPO.

### Definitions

The description that follows uses a number of terms that refer to recombinant DNA technology. In order to provide a clear and consistent understanding of the specification and the claims, the following definitions are provided.

Cloning Vector: A plasmid or phage DNA or other DNA sequence which is able to replicate autonomously in a host cell and which is characterized by one or a small number of restriction endonuclease recognition site. A foreign DNA fragment may be spliced into the vector at these sites in order to bring about the replication and cloning of the fragment. The vector may contain a marker suitable for use in the identification of transformed cells. For example, a marker may provide tetracycline resistance or ampicillin resistance.

Expression Vector: A vector similar to a cloning vector but which is capable of inducing the expression of the DNA that has been cloned into it after transformation into a host. The cloned DNA is usually placed under the control of (*i.e*., operably linked to) regulatory sequences such as promoters or enhancers. Promoter sequences may be constitutive, inducible or repressible.

Host: Any prokaryotic or eukaryotic cell that is the recipient of an expression vector or cloning vector is the "host" for that vector. Examples of cells that can serve as hosts are well known in the art as are techniques for cellular transformation (see *e.g.*, Sambrook, *et al.,* Molecular Cloning: A Laboratory Manual, 2nd ed., Cold Spring Harbor Press (1989)). Host cells may be present *in vivo* and undergo transfection of nucleic acids administered to an animal by injection.

Promoter: A DNA sequence typically found in the 5N region of a gene, located proximal to the start codon. Transcription is initiated at the promoter. If the promoter is of the inducible type, then the rate of transcription increases in response to an inducing agent.

Expression: Expression is the process by which a polypeptide is produced from DNA. The process involves the transcription of a DNA segment encoding the polypeptide into mRNA and the translation of this mRNA into the final polypeptide product.

### Detailed Description of the Invention

### Making of Expression Vectors

The present invention is directed to a method for treating anemia in dogs, cats and pigs by administering, respectively, expression vectors encoding canine, feline and porcine EPO. The full length amino acid sequences for these forms of EPO are shown in Figures 1 - 3. Wen, *et al,* (*Blood 82*:1507-1516 (1993)) describes procedures for PCR amplifying EPO from each of these species. MacLeod, *et al.* (*Am. J. Vet. Res. 59*:1144-1148 (1998)) also describes a procedure for isolating canine EPO and for incorporating it into an expression vector. Although these procedures may be used to obtain EPO sequences suitable for use in the present invention, many alternative techniques for isolating genetic elements have been described and can be used as well (*see e.g.,* Sambrook, *et al.,* Molecular Cloning: A Laboratory Manual, 2nd ed., Cold Spring Harbor Press (1989)). Thus, the DNA can be chemically synthesized, obtained by screening cDNA libraries, or amplified from cDNA with PCR probes corresponding to regions of the known EPO sequences. The preferred procedure is to chemically synthesize the desired EPO nucleotide sequence with a segment encoding a signal sequence at its 5N end. It is preferred that the signal sequence be homologous with the form of EPO being delivered (*e.g.*, the canine signal sequence should preferably be used with the canine hormone). However, other signal sequences may be used if desired, e.g., the canine signal sequence may be used with the porcine structural sequence. Restriction sequences should also be included 5N to the region encoding the signal sequence and 3N to the EPO structural sequence. These restriction sequences should be chosen to match corresponding sites in the vector that will be used for expressing EPO. In the case of the preferred VR1012 vector, the DNA may be constructed with an EcoRV site at its 5N end and a BgIII site at its 3N end.

Expression vectors may be constructed using standard techniques and should include a promoter operably linked to the DNA element coding for EPO. Transcription enhancers and other regulatory elements may also be present. Preferably, transcription is initiated at a CMV (cytomegalovirus) promoter. Examples of other promoters that may be used include that of the mouse metallothionein I gene (Haymer, *et al., J. Mol. Appl. Gen.* 1:273 (1982)); the TK promoter of herpes virus (McKnight, Cell 31:355-365 (1982)), and the SV40 early promoter (Benoist, *et al., Nature 290*:304 (1981)).

A large number of plasmids suitable for use in the present invention have been described (Botstein, *et al., Miami Winter Symp.* 19:265 (1982)); Broach, *Cell 28*:203 (1982); Bollon, *et al., J. Clin. Hematol. Oncol. 10*:39 (1980); and Maniatis, in Cell Biology: A Comprehensive Treatise, vol. 3, Academic Press, N.Y, pp. 563-608 (1980)). The preferred expression vector, "VR1012," may be constructed as described by Hartikka, *et al.* (*Hum. Gene Ther. 7*:1205-1217 (1996)).

### Chemical Form of Expression Vectors

A variety of different agents have been used to facilitate the uptake of DNA by cells *in vivo.* The present method is compatible with the use of any of these agents but, preferably, expression vectors will either be administered in a form free from transfection-facilitating agents or will be complexed with a cationic lipid. Suitable methods for preparing "naked" DNA are described in U.S. 5,703,055. Examples of cationic lipids that could be used are described in U.S. 5,264,68 and 5,459,127 and examples of cationic liposomes can be found in U.S. 4,897,355. When a transfection-facilitating agent is used, it should be chosen with the objective of maximizing cellular uptake of administered expression vectors without causing adverse side effects in the treated animal.

### Dosage Form and Route of Administration

Any route of administration that is compatible with the *in vivo* transfection of cells and subsequent expression of functional EPO may be used with the present method. Expression vectors encoding EPO may be administered as the sole active agent or in combination with other nucleic acids or therapeutically active drugs. Although intramuscular injection is preferred, other routes that may be used include intradermal, transdermal, intravenous, intraarterial, intraperitoneal, intracutaneous and subcutaneous routes. Vectors may also be delivered to animals by means of a "gene gun."

Expression vectors may be incorporated into pharmaceutical preparations made using methods that are standard in the art (see, *e.g.*, Remington's Pharmaceutical Sciences, 16th ed., A. Oslo editor, Easton PA (1980)). In general, preparations should be suitable for parenteral administration and will include aqueous solvents such as sterile isotonic saline, ethanol, polyglycols mixed with water, Ringer's solution, etc. Typically, the expression vector should constitute between about 0.01 mg/ml and 10 mg/ml of the final composition.

### Method of Treatment

An animal diagnosed as suffering from anemia should usually be administered a dose of between 0.01 mg and 20.0 mg by intramuscular injection in a volume of between about 0.5 and 30 ml. Single or multiple injections may be made. Thus, an animal given several injections may receive a total dosage of 100 mg or more. One regimen that has been found to be suitable involves making three injections of DNA into each hind limb of an animal, each injection being separated by a thirty minute interval. Effective treatment was accomplished by administering 2.5 mg of EPO DNA in 25 ml at each of the six injections.

These dosages are simply guidelines and the actual dosage selected for an individual animal will be determined by the attending veterinarian based upon clinical conditions. The effectiveness of a given dosage may be monitored at regular intervals, e.g., once every two weeks, by determining the hematocrit of the treated animal. It may be necessary to repeat administration every three or four months, with the frequency being adjusted based upon the response of an individual animal. Other factors that may influence dosage include the efficiency by which expression vectors are taken up by cells and the rate at which the promoter and other regulatory elements induce the synthesis of EPO after transfection.

The present method is compatible with any method of *in vivo* transfection that has been described in the art. The preferred procedures are those described in WO 90/11092 and in the Examples section herein. These, or similar procedures, may be effectively used to treat anemia in dogs and cats regardless of cause. Thus, animals should respond to the treatment procedure regardless of whether anemia is due to chronic renal failure, chemotherapy or simply old age.

### EXAMPLES

### Example 1: Preparation of DNA Encoding EPO

DNA sequences encoding the canine and feline forms of EPO were synthesized to correspond to the sequences shown in Figures 1 and 2 (including nucleotides encoding signal sequences). An EcoRV site was included at the 5N end of the synthesized DNA and a Bglll site was included at the 3N end. The DNA was then cloned into plasmid VR1012. This plasmid contains genetic elements, including a CMV promoter, that facilitate expression of cloned genes in mammalian cells both *in vitro* and *in vivo.* Recombinant VR1012 containing the canine EPO gene is referred to hereinafter as "canine EPO DNA," and recombinant VR1012 containing the feline EPO gene is termed "feline EPO DNA." Plasmids were extracted from *E. coli* cultures and purified using either cesium-chloride gradient ultracentrifugation or anion exchange chromatography. The quality of DNA preparations was determined by spectrophotometry and agarose gel electrophoresis.

### Example 2: Elevation of Mouse Hematocrits Following Injection With Canine EPO DNA

This study involved the use of three groups, each containing twenty mice. One group was treated with canine EPO DNA; a second with plasmid DNA without the canine EPO gene; and the third served as non-treated controls. Mice were given three injections of DNA in the rectus femoris muscle of each hind limb. Each injection contained 25 _{N}g of DNA in 50 µl of phosphate buffered saline (PBS) with administrations being separated by thirty minute intervals. Hematocrits were determined prior to the first treatment on day 0 and on days 9, 16, 23 and 36 post-treatment. Blood was collected by retro-orbital venous puncture using a heparinized hematocrit tube. The blood tubes were centrifuged and the hematocrit was determined with a Micro-Hematocrit capillary tube reader. The data collected was

**Table 1:**

| **Statistical Pairwise Analysis of Mice Treated With Canine EPO DNA** | | | | | |
|---|---|---|---|---|---|
| | Day 0 | Day 9 | Day 16 | Day 23 | Day 36 |
| plasmid control vs. canine EPO | 0.1442 | 0.0001 | 0.0001 | 0.0001 | 0.1502 |
| plasmid control vs. non-injected control | 0.0287 | 0.0563 | 0.2141 | 0.9889 | 0.7015 |
| canine EPO vs. non-injected control | 0.4502 | 0.0001 | 0.0001 | 0.0001 | 0.0665 |

Pairwise analysis of the data showed a significant elevation (P value of less than 0.05) in mice treated with canine EPO DNA up to day 36 post-treatment compared to hematocrits in mice of the non-injected control group. Comparison of mice receiving plasmid DNA without the canine EPO gene and mice that were not injected showed a significant difference at day 0. This was considered to be an experimental anomaly since the animals were not yet treated when the blood was collected. There was no significant difference between the two control groups at any other time point. A decrease in hematocrits of canine EPO DNA-treatment mice was observed two weeks post-treatment and may be attributable to the induction of an immune response to canine EPO in the mice.

### Example 3: Elevation of Hematocrits in Mice Injected With Feline EPO DNA

Two groups of 5 mice each were treated with either feline EPO DNA or just solvent (PBS, "non-treated control"). The concentrations, volumes and injection regiment were as described in Example 2. Hematocrits were determined prior to treatment on day 0 and on days 7, 14 and 21 post-treatment. Results were analyzed statistically and are shown in Table 2.

**Table 2:**

| **Statistical Pairwise Analysis of Mice Administered Feline EPO DNA** | | | | |
|---|---|---|---|---|
| | Day 0 | Day 7 | Day 14 | Day 22 |
| feline EPO vs. non-injected Control | 0.9357 | 0.0456 | 0.0001 | 0.0111 |

Pairwise analysis of the data as shown in Table 2 indicates that there was a significant elevation of hematocrits in mice treated with feline EPO DNA compared to the nontreated control group on days 7, 14 and 21 post-treatment.

### Example 4: Elevation of Hematocrits in Dogs Injected With Canine EPO DNA

In order to estimate the amount of canine EPO DNA required to induce an elevation of hematocrits in dogs, animals were divided into three experimental groups. Each group received six injections following the general procedure described in Example 2. The volume and amount used at each injection are shown in Table 3.

**Table 3:**

| **Conditions for Experimental Groups in Study of Dogs Injected With EPO DNA** | | | | |
|---|---|---|---|---|
| Experimental Group | No. of Dogs In Study | Volume | Amount of DNA | Designation |
| Group A | 4 | 25 ml | 7.5 mg | hi vol, hi dose |
| Group B | 4 | 25 ml | 2.5 mg | hi vol, low dose |
| Group C | 4 | 3 ml | 2.5 mg | low vol, low dose |

Each group contained four dogs, two dogs treated with canine EPO DNA and two controls treated with plasmid DNA containing the human soluble embryonic alkaline phosphatase (SEAP) gene. Hematocrits were measured as described previously.

Administration of canine EPO DNA lead to an elevation of hematocrits that was sustained for greater than thirty-two days in all groups whereas administration of SEAP DNA had no effect on hematocrits. Although the relatively small sample size limited statistical analysis, the percentage increase in hematocrit levels in all groups was considered to be physiologically relevant. The data indicated that (i) administration of canine EPO DNA leads to a rise in hematocrits in dogs, validating the concept of gene therapy, and (ii) the elevation of hematocrits is sustained for an extended period of time, suggesting that the treated dogs did not develop an immune response to EPO.

In terms of individual animals, both dogs in the "hi vol, low dose" group (administered a total of 15 mg in 150 ml) group responded with an elevation in hematocrits. In each of the "hi vol, hi dose" and "low vol, low dose" groups, the response of one of the two animals in the group was superior to the other. When the poor responders were treated with a "booster" dose of canine EPO DNA under "hi vol, low dose" conditions, both dogs responded with an elevation in hematocrits. This suggests that there may be greater consistency in hematocrit responses when dogs are administered canine EPO DNA under "hi vol, low dose" conditions.

### Example 5: Evaluation of Rate of Hematocrit Recovery in Nephrectomized and Phlebotomized Dogs

The effect of canine EPO DNA administration on the rate of recovery from anemia was examined in an experimental model of renal failure. Sixteen healthy beagle dogs were anesthetized and the renal arteries, veins and ureter of their left kidney were ligated and severed. The kidney was then mobilized and removed. The renal artery of the right kidney was ligated and severed at the first bifurcation leaving approximately half of the remaining kidney normally perfused. Dogs were allowed to recover at least one week before phlebotomy. In order to induce anemia, the nephrectomized dogs were bled three times at twenty-four hour intervals. At each scheduled phlebotomy, a blood volume equal to two percent of body weight was removed and immediately replaced with twice the volume of lactated Ringer's solution. Canine EPO DNA was administered to twelve dogs in the manner described previously ("hi vol, low dose" conditions). Six dogs were administered naked canine EPO DNA and six dogs were administered canine EPO DNA complexed with lipid (Lipofectamine□, Life Technologies, Inc., Gaithersburg, MD) at a ratio of 4 mg DNA/3 ml Lipofectamine. Four dogs were left untreated following nephrectomy and phlebotomy.

All nephrectomized and phlebotomized dogs exhibited elevated hematocrits from the values recorded immediately following phlebotomy. Three of the four dogs in the control group, (*i.e*., the dogs not treated with canine EPO DNA) exhibited elevated hematocrits that stabilized below the base line values recorded for the dogs prior to nephrectomy and phlebotomy. In contrast, the hematocrits of the dogs treated with canine EPO DNA rose and stabilized at values comparable to those observed prior to nephrectomy and phlebotomy. This suggests that the hematocrit rise in these dogs is due to EPO production from the administered DNA. Overall, the rate of hematocrit rise was superior in dogs treated with Lipofectamine-complexed DNA than in either dogs treated with naked DNA or untreated dogs. Consistent with these observations, reticulocyte counts were higher in Lipofectamine-complexed canine EPO DNA treated dogs than in controls, indicating elevated erythropoiesis. These results suggest that EPO from the administered DNA induced both a higher hematocrit and a faster elevation in anemic dogs. Although the rate of recovery from anemia was comparable between dogs treated with naked canine EPO DNA and in dogs left untreated, the results of the study taken as a whole indicate that EPO gene therapy can be successfully used in dogs.

### Example 6: Elevation of Hematocrit in Outbred Strain of Mice (CD1) Following Injection With Canine EPO DNA

In examples 2 and 3 described above, mice from an inbred strain, BaIB/C, were injected with DNA in a volume of 50 ul. In the present example, canine EPO DNA was administered in a volume of 25 ul to mice in an outbred strain, CD1. Some injections were made using sodium phosphate as the solvent (150 mM pH 7.2-7.4) and others were made in PBS (Dulbecco's phosphate buffered saline, Life Technologies, Inc., Gaithersburg, MD).

**Table 4:**

| **Conditions for Experimental Groups in Study of Outbred Mice Injected With EPO DNA** | | | | |
|---|---|---|---|---|
| Group | Amount of DNA (ug) per hind limb | Vol (ul) per injection | Solvent | # of injections per hind limb |
| I | 100 | 25 | sodium phosphate | 1 |
| II | 50 | 25 | sodium phosphate | 1 |
| III | 25 | 25 | sodium phosphate | 1 |
| IV | 100 | 25 | PBS | 1 |
| V | 50 | 25 | PBS | 1 |
| VI | 25 | 25 | PBS | 1 |
| VII (positive control) | 100 | 50 | PBS | 3 |
| VIII (negative control) | 0 | 25 | sodium phosphate | 1 |

The results obtained suggested that DNA-based EPO gene therapy can be successfully used in an outbred mammalian population. It was also found that DNA in sodium phosphate' elicits a superior hematocrit response relative to DNA in PBS and that administration of DNA in a volume of 25 ul using 1 injection per hind limb is sufficient to induce a significant elevation of hematocrits.

### Example 7: Elevation of Hematocrit in "Geriatric" Mice Following Injection With Canine EPO DNA

This study involved a demonstration of elevation of hematocrit in "geriatric" (retired breeders) Balb/C mice.

**Table 5:**

| **Conditions for Experimental Groups in Study of Geriatric Mice Injected With EPO DNA** | | | | |
|---|---|---|---|---|
| Group | Amount of DNA (ug) per hind limb | Vol (ul) per injection | Solvent | # of injections per hind limb |
| I | 100 | 25 | sodium phosphate | 1 |
| II (negative control) | 0 | 25 | sodium phosphate | 1 |

The results obatined suggest that DNA-based EPO gene therapy can be successfully used in treating a "geriatric" mammalian population.

### Example 8: Elevation of Hematocrit in Cats Following Injection With Feline EPO DNA

Cats beween 6 and 8 months of age were injected, splenectomized at day -25, and treated with feline EPO DNA according to the regimen described below. Plasmid DNA was taken up in sodium phosphate as descibed above and injected intramuscularly into the rectus femoris muscle. For a negative control, cats were injected with 150 mM sodium phosphate, pH 7.2-7.4. There were 5 cats in each group.

**Table 6:**

| **Conditions for Experimental Groups in Study of Cats Injected With EPO DNA** | | | | |
|---|---|---|---|---|
| Group | Amount of DNA (ug) per animal | Conc. of DNA (mg/ml) | Total vol. per injection | # of injections per hind limb |
| 1 (negative control) | 0 | 0 | 15 | 1 |
| 2 | 37.5 | 0.5 | 15 | 3 |
| 3 | 12.5 | 0.5 | 12.5 | 1 |
| 4 | 5 | 0.5 | 5 | 1 |
| 5 | 1 | 0.1 | 5 | 1 |
| 6 | 20 | 2 | 5 | 1 |

The results obtained suggest that plasmid DNA-based EPO gene therapy may be successfully used in cats. All EPO DNA treatments led to a significant elevation of hematocrits. In terms of dosage, it was found that 1 mg of DNA at a concentration of 0.1 mg/ml administered in 10 ml sodium phosphate in 2 equal volume injections, 1 per hind limb, elicited a significant elevation of hematocrits when compared to untreated cats.

## Claims

1. A method of treating anemia in a dog comprising administering an expression vector to said dog wherein:
(a) said expression vector comprises a distinct EPO sequence element consisting essentially of nucleotides encoding canine erythropoietin;
(b) said expression vector further comprises a promoter element operably linked to said EPO sequence element; and
(c) said expression vector is administered at a dosage sufficient to cause a statistically significant increase in the hematocrit of said dog.

2. The method of claim 1, wherein said expression vector is administered in a form free from transfection-facilitating agents.

3. The method of claim 1, wherein said expression vector is complexed with a cationic lipid.

4. The method of claim 1, wherein said expression vector is administered as part of a liposome.

5. The method of claim 1, wherein said vector is administered at a concentration of between about 0.01 mg/ml and 10 mg/ml.

6. The method of claim 1, wherein said expression vector is administered at a concentration of about 0.1 mg/ml.

7. The method of any one of claims 1-6, wherein said vector is administered by means of intramuscular injection.

8. A pharmaceutical composition for parenteral administration, comprising:
(a) an expression vector wherein:
i) said expression vector comprises a distinct EPO sequence element consisting essentially of nucleotides encoding canine erythropoietin;
ii) said expression vector further comprises a promoter element operably linked to said EPO sequence element; and
(b) an aqueous solvent in which said expression vector is dissolved or suspended.

9. The pharmaceutical composition of claim 8, wherein said expression vector is present at a concentration of between about 0.01 mg/ml and 10 mg/ml.

10. The pharmaceutical composition of claim 8, wherein said expression vector is present at a concentration of about 0.1 mg/ml.

11. A method of treating anemia in a cat comprising administering an expression vector to said cat wherein:
(a) said expression vector comprises a distinct EPO sequence element consisting essentially of nucleotides encoding feline erythropoietin;
(b) said expression vector further comprises a promoter element operably linked to said EPO sequence element; and
(c) said expression vector is administered at a dosage sufficient to cause a statistically significant increase in the hematocrit of said cat.

12. The method of claim 11, wherein said expression vector is administered in a form free from transfection-facilitating agents.

13. The method of claim 11, wherein said expression vector is complexed with a cationic lipid.

14. The method of claim 11, wherein said expression vector is administered as part of a liposome.

15. The method of claim 11, wherein said vector is administered at a concentration of between about 0.01 mg/ml and 10 mg/ml.

16. The method of claim 11, wherein said expression vector is administered at a concentration of about 0.1 mg/ml.

17. The method of any one of claims 11-16, wherein said vector is administered by means of intramuscular injection.

18. A pharmaceutical composition for parenteral administration, comprising:
(a) an expression vector wherein:
i) said expression vector comprises a distinct EPO sequence element consisting essentially of nucleotides encoding feline erythropoietin;
ii) said expression vector further comprises a promoter element operably linked to said EPO sequence element; and
(b) an aqueous solvent in which said expression vector is dissolved or suspended.

19. The pharmaceutical composition of claim 18, wherein said expression vector is present at a concentration of between about 0.01 mg/ml and 10 mg/ml.

20. The pharmaceutical composition of claim 18, wherein said expression vector is present at a concentration of between about 0.01 mg/ml.

21. A method of treating anemia in a pig comprising administering an expression vector to said pig wherein:
(a) said expression vector comprises a distinct EPO sequence element consisting essentially of nucleotides encoding porcine erythropoietin;
(b) said expression vector further comprises a promoter element operably linked to said EPO sequence element; and
(c) said expression vector is administered at a dosage sufficient to cause a statistically significant increase in the hematocrit of said pig.

22. The method of claim 21, wherein said expression vector is administered in a form free from transfection-facilitating agents.

23. The method of claim 21, wherein said expression vector is complexed with a cationic lipid.

24. The method of claim 21, wherein said expression vector is administered as part of a liposome.

25. The method of claim 21, wherein said vector is administered at a concentration of between about 0.01 mg/ml and 10 mg/ml.

26. The method of claim 21, wherein said expression vector is administered at a concentration of about 0.1 mg/ml.

27. The method of any one of claims 21-26, wherein said vector is administered by means of intramuscular injection.

28. A pharmaceutical composition for parenteral administration, comprising:
(a) an expression vector wherein:
i) said expression vector comprises a distinct EPO sequence element consisting essentially of nucleotides encoding porcine erythropoietin;
ii) said expression vector further comprises a promoter element operably linked to said EPO sequence element; and
(b) an aqueous solvent in which said expression vector is dissolved or suspended.

29. The pharmaceutical composition of claim 28, wherein said expression vector is present at a concentration of between about 0.01 mg/ml and 10 mg/ml.

30. The pharmaceutical composition of claim 28, wherein said expression vector is present at a concentration of about 0.1 mg/ml.
